# EUROPEAN PATENT SPECIFICATION

(11) **EP 3 971 183 B1**
(45) Date of publication and mention of the grant of the patent: **29.07.2026**
(21) Application number: 20826049.7
(22) Date of filing: 18.06.2020
(51) Int. Cl.: C07D 209/08

(54) **METHOD FOR PREPARING INDOLE OR INDAZOLE COMPOUND**
VERFAHREN ZUR HERSTELLUNG VON INDOL- ODER INDAZOLVERBINDUNGEN
PROCÉDÉ DE PRODUCTION D'UN COMPOSÉ D'INDOLE OU D'INDAZOLE

(30) Priority: 19.06.2019 KR 20190073017
(43) Date of publication of application: 23.03.2022
(73) Proprietor: LG Chem, Ltd., Seoul 07336 (KR)
(72) Inventor: LEE, Sang Dae, Daejeon 34122 (KR); PARK, Ae Ri, Daejeon 34122 (KR); CHOI, Bo Seung, Daejeon 34122 (KR); KIM, Bong Chan, Daejeon 34122 (KR)
(74) Representative: Hoffmann Eitle
(86) International application number: PCT/KR2020/007900
(87) International publication number: WO 2020/256430

(56) References cited:
- EP-A1- 3 037 418
- WO-A1-2010/111058
- KR-A- 20010 031 849
- KR-A- 20090 075 638
- KR-A- 20130 056 244
- KR-A- 20130 087 283
- KR-A- 20150 022 707

## Description

### TECHNICAL FIELD

### Cross-reference to Related Application

This application claims the benefit of Korean Patent Application No. 10-2019-0073017, filed on June 19, 2019, in the Korean Intellectual Property Office.

### Technical Field

The present invention relates to a method for preparing an indole or indazole compound containing a nitro group, which is an intermediate structure necessary for the synthesis of a pharmaceutically useful indole or indazole compound.

### BACKGROUND ART

Many results of studies on compounds having an indole structure as a parent nucleus have been reported, and representative examples include, for example, PCT International Publication No. WO2006/112549, which reported that the compounds have the activity for the glucokinase, PCT International Publication No. WO1995/007276, which reported that the compounds are useful as anti-tumor agents and inhibitors against the production of cardiovascular system, and PCT International Publication No. WO2004/018428, which reported that the compounds can be used as antibiotics.

Among these, the present invention relates to a method for preparing an indole or indazole compound containing a nitro group, as an intermediate structure necessary for obtaining an indole or indazole compound exhibiting an effect of prevention or treatment and amelioration for cellular necrosis and necrosis-associated diseases.

Conventionally, since an intermediate containing an ether group is synthesized during the synthesis of an indole or indazole compound intermediate containing a nitro group from a benzoic acid containing a nitro group, there is a limitation in that the reactivity is low when a halogenated reaction, an acetylene intermediate forming reaction, and a cyclization reaction are performed from a benzoic acid, which is a starting material, so that a side reaction ratio is high, and a column purification process such as filtration purification using celite must be performed for each step in order to remove by-products due to the side reaction. As such, there are limitations in that when the column purification process is performed, each synthesis step is not performed in situ in the same reactor, and thus process efficiency is deteriorated, and the column purification process is difficult to scale up, and thus cannot be applied to a process for mass production.

In addition, there is a limitation in that when an intermediate containing an ether group is used, a process of substituting the ether group with a carboxylic acid is necessarily carried out, and thus the process steps become complicated.

Accordingly, studies on a process for preparing an intermediate structure necessary for obtaining an indole or indazole compound exhibiting an effect of prevention or treatment and amelioration for cellular necrosis and necrosis-associated diseases and increasing the efficiency of the process are urgently needed.

Furthermore, EP 3 037 418 A1 discloses a specific indole compound, a pharmaceutically acceptable salt or isomer thereof, a composition for prevention or treatment of necrosis and necrosis-associated diseases, and a method for preparing the composition, the composition comprising the indole compound or the pharmaceutically acceptable salt or isomer thereof as an active ingredient. Finally, KR 2009/0075638 A discloses an indole and indazole derivative which is said to have preservative effect of cell, tissue and organ to prevent reperfusion injury generated after transplantation or preservation at low temperature.

### [PRIOR ART DOCUMENTS]

### [PATENT DOCUMENTS]

PCT International Publication No. WO2006/112549 (October 26, 2006)
PCT International Publication No. WO1995/007276 (March 16, 1995)
International Publication No. WO2004/018428 (March 4, 2004)

### DISCLOSURE OF THE INVENTION

### TECHNICAL PROBLEM

An aspect of the present invention provides a method for preparing an indole or indazole compound containing a nitro group from a benzoic acid compound containing a nitro group, as an intermediate structure necessary for obtaining the indole or indazole compound exhibiting an effect of prevention or treatment and amelioration for cellular necrosis and necrosis-associated diseases, wherein the method can simplify a complex process of six or more steps in the related art, increase the reactivity of each intermediate step reaction to increase the yield of the product, and replace column purification with solid purification, thereby being applied to a mass production process.

### TECHNICAL SOLUTION

According to an aspect of the present invention, there is provided a method for preparing a compound represented by Formula 1, the method including
a) adding a halogen donor to a compound represented by Formula 2 to prepare a compound represented by Formula 3, wherein the halogen donor is N-iodosuccinimide;
b) adding a compound represented by Formula 4 below in the presence of a metal catalyst after Step a) above;
c) adding a base in the presence of the metal catalyst to prepare a compound represented by Formula 5 below;
d) adding a reducing agent to the compound represented by Formula 5 to prepare the compound represented by Formula 1: 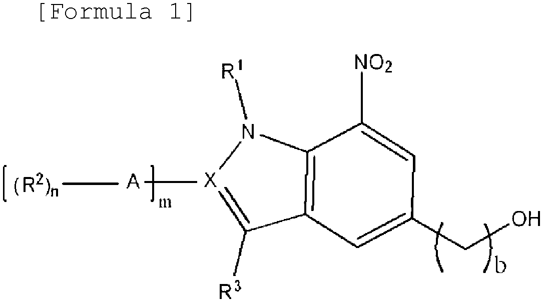 wherein, in Formula 1 above,
   n is an integer of 1 to 3,
   m is 1,
   b is an integer of 1 to 3,
   A represents phenyl, or 5-membered heteroaryl or heterocycle which each contains 1 to 3 heteroatoms selected from among N, O and S atoms and is optionally substituted by R, wherein R represents hydrogen or C₁-C₄-alkyl optionally substituted by hydroxy or amino,
   X represents C,
   R¹ represents hydrogen, C₁-C₆-alkyl or -(CH₂)ᵣNR⁸R⁹, wherein r is an integer of 2 to 5, R⁸ and R⁹ each independently represent hydrogen or C₁-C₃-alkyl,
   R² represents hydrogen, halogen or C₁-C₆-alkoxy, or represents - (CH₂)ₚC(O)₂R⁸, - (CH₂)ₚOR⁸, - (CH₂)ₚNR^{B}R⁹, -NHR¹⁰, - N (H) S (O)₂R⁸, or -NHC (O)₂R¹⁰, or represents -(CH₂)ₚ-heterocycle-R¹⁰ in which the heterocycle moiety is a 5-6 membered ring containing one or two heteroatoms selected from among N, O, and S atoms, wherein p is an integer of 0 to 3, R⁸ and R⁹ are as defined above, and R¹⁰ represents hydrogen, oxo, C₁-C₆-alkylcarbonyl, C₁-C₆-alkoxy or C₁-C₆- alkyl, or a 5-6 membered heterocycle containing one or two nitrogen atoms as a heteroatom, and
   R³ represents hydrogen, halogen, C₁-C₆-alkyl or phenyl, or represents -(CH₂)_{q}-heterocycle in which the heterocycle moiety is a 5-6 membered ring containing one or two heteroatoms selected from among N and O atoms, wherein q is an integer of 1 to 3,
   wherein alkyl, alkoxy, aryl, cycloalkyl, heterocycle and heteroaryl may be optionally substituted, and the substituents are one or more selected from the group consisting of hydroxy, C₁-C₆-alkylamino, di (C₁-C₆-alkyl) amino, carboxy, C₁-C₆-alkyl, C₁-C₆-alkoxy, carboxy-C₁-C₆-alkyl and oxo, 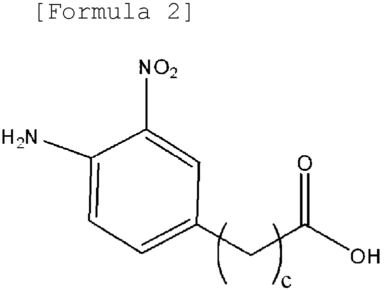
   wherein, in Formula 2 above,
   c is an integer of 0 to 2, 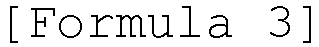 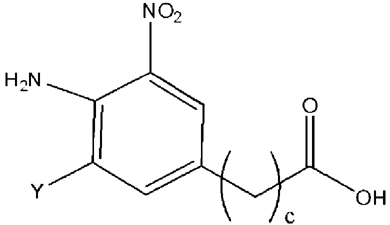
   wherein, in Formula 3 above, Y iodo, and
   c is the same as defined in Formula 2 above, 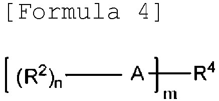
   wherein, in Formula 4 above,
   R², A, n and m are the same as defined in Formula 1, and
   R⁴ is acetylene 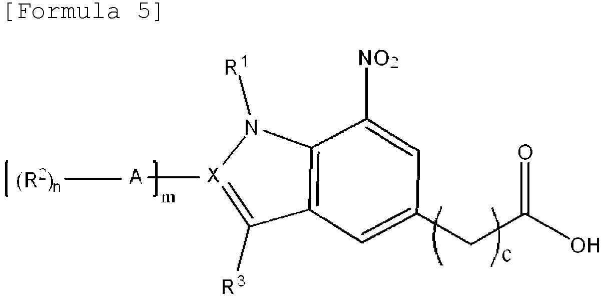
   wherein in Formula 5 above, R¹ to R³, A, X, n and m are the same as defined in Formula 1 above, and c is the same as defined in Formula 2 above.

According to another aspect of the present invention, there is provided a method for preparing a compound represented by Formula 6, the method including preparing a compound represented by Formula 1 above; and synthesizing the compound represented by Formula 6 below from the compound represented by Formula 1: 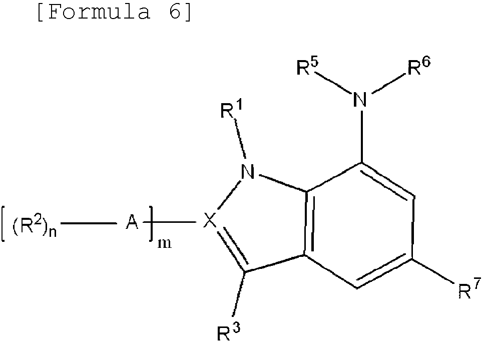 wherein, in Formula 6 above,
R¹, R², R³, n, and m are the same as defined in Formula 1 above,
R⁵ represents hydrogen, C₁-C₆-alkyl, C₃-C₆-cycloalkyl, heterocycle or heterocyclyl-C₁-C₆-alkyl, wherein the heterocycle is a 3-8 membered ring containing one to three heteroatoms selected from among N and O atoms,
R⁶ represents -(CR⁸R⁹)u-Z-D-W-R¹⁴, wherein u is an integer of 0 to 3, Z represents a direct bond or is selected from the group consisting of -C(O)- and -C(0)0-, D represents a direct bond, C₄-C₆-cycloalkyl, 5-6 membered heteroaryl containing one or two N atoms, or a 5-6 membered heterocycle containing one or two heteroatoms selected from among N, O and S atoms, W represents a direct bond, or -NR^{B}-,-C(O)-, - C(O)O-, -C(O)NR¹²- or -S(O)ₜ-, R¹² represents hydrogen, C₁-C₃-alkyl or C₆-C₁₀-aryl, t is an integer of 1 or 2, and R¹⁴ represents hydrogen, hydroxy, C₁-C₆-alkyl, a 5-6 membered heterocycle containing one to three heteroatoms selected from among N, 0 and S atoms, or C₆-C₁₀-Ar-C₁-C₆-alkyl, and
R⁷ represents -Y'R¹¹, wherein Y' is a direct bond or represents -(CR⁸R⁹)ₕY"-, wherein h is an integer of 0 to 3, R¹¹ is selected from the group consisting of hydrogen, halogen, C₁-C₆-alkyl and -(CH₂)ᵥB-R¹³, v is an integer of 0 to 3, B represents a 5-6 membered heterocycle containing one or two heteroatoms selected from among N, O, and S atoms, or represents C₆-C₁₀-aryl, R¹³ represents hydrogen, cyano, halogen, hydroxy, oxo, thiol, carboxy or carboxy-C₁-C₆-alkyl, R⁸ and R⁹ are as defined in claim 1, and Y" is selected from the group consisting of -O-, -C(O)-, and -C(O)O-,
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo.

### ADVANTAGEOUS EFFECTS

The method for preparing an indole or indazole compound of the present invention can increase the yield of the product by simplifying a process and increasing the reactivity of each intermediate step, and can greatly improve the efficiency of the process since a column purification process using celite can be omitted due to reducing the ratio of by-products by improving the reactivity of a target reaction for each step, compared to a conventional method for preparing an indole or indazole compound containing a nitro group, and can be easily applied to a mass production process by replacing the column purification process with a solid purification process.

### MODE FOR CARRYING OUT THE INVENTION

Hereinafter, the present invention will be described in more detail to aid in understanding the present invention.

In the definition for the substituents of the compound of Formula (1) according to the present invention, the term "alkyl" refers to an aliphatic hydrocarbon radical. Alkyl may be "saturated alkyl" which does not include an alkenyl or alkynyl moiety, or "unsaturated alkyl" which includes at least one alkenyl or alkynyl moiety. The term "alkenyl" refers to a group containing at least one carbon-carbon double bond, and the term "alkynyl" refers to a group containing at least one carbon-carbon triple bond Alkyl may be branched or linear when used alone or in a composite form such as alkoxy.

The alkyl group may have 1 to 20 carbon atoms unless otherwise defined. The alkyl group may be medium-sized alkyl having 1 to 10 carbon atoms. The alkyl group may be lower alkyl having 1 to 6 carbon atoms. A typical alkyl group includes, but is not limited to, methyl, ethyl, propyl, isopropyl, butyl, isobutyl, t-butyl, pentyl, hexyl, ethenyl, propenyl, butenyl, etc. For example, C₁-C₄-alkyl has 1 to 4 carbon atoms in the alkyl chain, and is selected from the group consisting of methyl, ethyl, propyl, iso-propyl, n-butyl, iso-butyl, sec-butyl and t-butyl.

The term "alkoxy" refers to alkyloxy having 1 to 10 carbon atoms unless otherwise defined.

The term "cycloalkyl" refers to a saturated aliphatic 3-10 membered ring unless otherwise defined. A typical cycloalkyl group includes, but is not limited to, cyclopropyl, cyclobutyl, cyclopentyl, cyclohexyl, etc.

The term "aryl" includes at least one ring having a covalent π electron system, for example, a monocyclic or fused polycyclic (i.e., rings that share adjacent pairs of carbon atoms) group. That is, unless otherwise defined herein, aryl refers to an aromatic 4-10 membered, preferably 6-10 membered, monocyclic or multicyclic ring including phenyl, naphthyl, etc.

The term "heteroaryl" refers to an aromatic 3-10 membered ring, preferably 4-8 membered ring, more preferably 5-6 membered ring, which contains 1 to 3 heteroatoms selected from the group consisting of N, O and S and may be fused with benzo or C₃-C₈ cycloalkyl unless otherwise defined. Examples of monocyclic heteroaryl include thiazole, oxazole, thiophene, furan, pyrrole, imidazole, isoxazole, isothiazole, pyrazole, triazole, triazine, thiadiazole, tetrazole, oxadiazole, pyridine, pyridazine, pyrimidine, and pyrazine. Examples of bicyclic heteroaryl include indole, indoline, benzothiophene, benzofuran, benzimidazole, benzoxazole, benzisoxazole, benzthiazole, benzthiadiazole, benztriazole, quinoline, isoquinoline, purine, and furopyridine.

The term "heterocycle" refers to a 3-10 membered ring, preferably 4-8 membered ring, more preferably 5-6 membered ring, which contains 1 to 3 heteroatoms selected from the group consisting of N, O and S and may be fused with benzo or C₃-C₈ cycloalkyl, and is saturated or contains 1 or 2 double bonds, unless otherwise defined. Examples of the heterocycle include pyrroline, pyrrolidine, imidazoline, imidazolidine, pyrazoline, pyrazolidine, pyran, piperidine, morpholine, thiomorpholine, piperazine, and hydrofuran.

Unless otherwise defined, terms and abbreviations used herein may be interpreted as meanings commonly understood by those skilled in the art to which the present invention pertains.

The present invention relates to a method for preparing an indole or indazole compound containing a nitro group represented by Formula 1 below, which is an intermediate structure necessary for synthesizing a pharmaceutically useful indole or indazole compound, the method being characterized by comprising
a) adding a halogen donor to a compound containing benzoic acid represented by Formula 2 below to prepare a compound represented by Formula 3, wherein the halogen donor is N-iodosuccinimide;
b) adding a compound represented by Formula 4 below in the presence of a metal catalyst after Step a) above;
c) adding a base in the presence of the metal catalyst to prepare a compound represented by Formula 5 below;
d) adding a reducing agent to the compound represented by Formula 5 to prepare the compound represented by Formula 1: 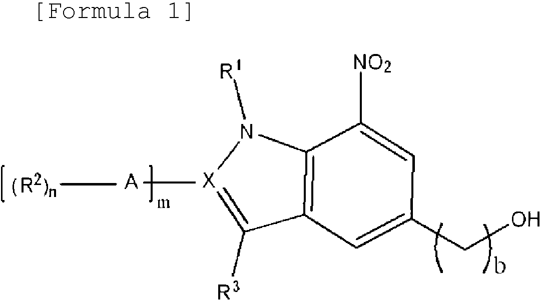 wherein, in Formula 1 above,
   n is an integer of 1 to 3, and
   m is 1,
   b is an integer of 1 to 3,
   A represents phenyl, or 5-membered heteroaryl or heterocycle which each contains 1 to 3 heteroatoms selected from among N, O and S atoms and is optionally substituted by R, wherein R represents hydrogen or C₁-C₄-alkyl optionally substituted by hydroxy or amino,
   X represents C,
   R¹ represents hydrogen, C₁-C₆-alkyl or -(CH₂)ᵣNR⁸R⁹, wherein r is an integer of 2 to 5, R⁸ and R⁹ each independently represent hydrogen or C₁-C₃-alkyl,
   R² represents hydrogen, halogen or C₁-C₆-alkoxy, or represents -(CH₂)ₚC(O)₂R⁸, - (CH₂)ₚOR⁸, - (CH₂)ₚNR^{B}R⁹, -NHR¹⁰, - N (H) S (O)₂R⁸, or -NHC (O)₂R¹⁰, or represents -(CH₂)ₚ-heterocycle-R¹⁰ in which the heterocycle moiety is a 5-6 membered ring containing one or two heteroatoms selected from among N, O, and S atoms, wherein p is an integer of 0 to 3, R⁸ and R⁹ are as defined above, and R¹⁰ represents hydrogen, oxo, C₁-C₆-alkylcarbonyl, C₁-C₆-alkoxy or C₁-C₆- alkyl, or a 5-6 membered heterocycle containing one or two nitrogen atoms as a heteroatom, and
   R³ represents hydrogen, halogen, C₁-C₆-alkyl or phenyl, or represents -(CH₂)_{q}-heterocycle in which the heterocycle moiety is a 5-6 membered ring containing one or two heteroatoms selected from among N and O atoms, wherein q is an integer of 1 to 3,
   wherein alkyl, alkoxy, aryl, cycloalkyl, heterocycle and heteroaryl may be optionally substituted, and the substituents are one or more selected from the group consisting of hydroxy, C₁-C₆-alkylamino, di (C₁-C₆-alkyl) amino, carboxy, C₁-C₆-alkyl, C₁-C₆-alkoxy, carboxy-C₁-C₆-alkyl and oxo, 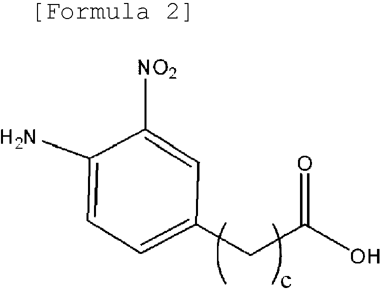
   wherein, in Formula 2 above,
   c is an integer of 0 to 2, 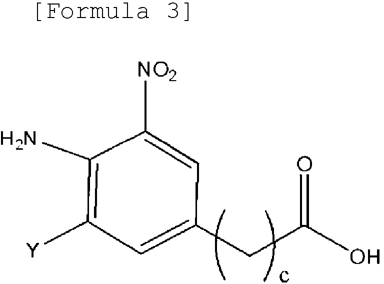
   wherein, in Chemical Formula 3, Y is iodo, and
   c is the same as defined in Formula 2 above 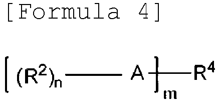
   wherein, in Formula 4 above,
   R², A, n and m are the same as defined in Formula 1, and
   R⁴ is acetylene 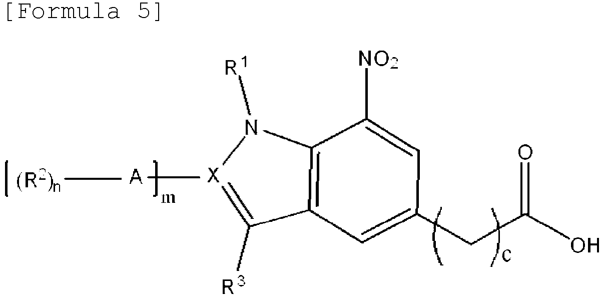
   wherein in Formula 5 above, R¹ to R³, A, X, n and m are the same as defined in Formula 1 above, and c is the same as defined in Formula 2 above.

Specifically, in Formula 1 above, R¹ above may be hydrogen, C₁-C₆-alkyl, or di(C₁-C₃-alkyl)amino-C₂-C₃-alkyl.

In addition, R² above may represent hydrogen, halogen, carboxy, carboxy-C₁-C₃-alkyl, C₁-C₃-alkoxycarbonyl, C₁-C₃-alkoxycarbonyl-C₁-C₃-alkyl, hydroxy-C₁-C₃-alkyl optionally substituted by one oxo group, C₁-C₃-alkoxy, -(CH₂)ₚNR^{B}R⁹, - NHR¹⁰, -N(H)S(O)₂R¹⁰ or -NHC(O)₂R¹⁰ or may be -(CH₂)ₚ-heterocycle-R¹⁰, wherein heterocycle, p, R⁸, R⁹ and R¹⁰ are as defined above.

Further, R³ may represent hydrogen, methyl or halogen, or may represent phenyl optionally substituted by C₁-C₃-alkoxy, or may be heterocyclyl-C₁-C₃-alkylene in which the heterocycle moiety is a 5-6 membered ring containing one or two heteroatoms selected from N and O atoms and optionally substituted with one or two oxo groups.

In addition, b above may be specifically 1 to 2, more specifically 1.

Moreover, in Formula 2 above, c may specifically have a value of b-1, more specifically 0 to 1, and still more specifically 0.

Further, in Formula 3 above, Y may be specifically bromo or iodo, and more specifically, iodo, and c is the same as defined in Formula 2 above.

In addition, the compound represented by Formula 1 above may be represented by Formula (1a) below: 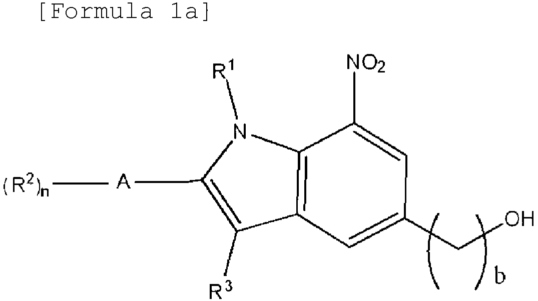 wherein, in the Formula above, n, A, R¹, R², R³ and b are as defined in claim 1.

For example, the compound represented by Formula 1 or Formula 1a above may be (7-nitro-2-phenyl-1H-indol-5-yl)methanol, and the compound represented by Formula 2 above may be 4-amino-3-nitrobenzoic acid.

In addition, Step a) above may be carried out in the presence of an acid catalyst, wherein the acid catalyst may include at least one among sulfuric acid, nitric acid, acetic acid, trifluoroacetic acid, and hydrochloric acid, and preferably sulfuric acid.

In addition, Step a) above may be adding the halogen donor to the compound represented by Formula 2 in the presence of the acid catalyst at a temperature of 0-25 °C, and after the addition, the halogenated reaction of the compound represented by Formula 2 may be carried out by raising the temperature to 60-120 °C and then stirring the mixture for 2-10 hours.

Step b) of the present invention may be a linking reaction for joining a halogenated benzoic acid compound with an acetylene compound represented by formula 4 above, and specifically, Step b) above may be performed in the presence of a metal catalyst, wherein the metal catalyst may contain a metal such as Cu(I), Pd(II), and the metal catalyst may be specifically a compound in the form of a halide salt containing the metal. For example, CuI and Pd(PPh₃)₂Cl₂ may be used as the metal catalyst.

Further, Step b) above may be performed at a temperature of 30-100 °C for 2-10 hours.

In addition, according to an embodiment of the present invention, after step b), a step (Step c) for adding a base in the presence of a metal catalyst to prepare a compound represented by Formula 5 below is further included, wherein Step b) and Step c) above may be performed in the same reactor (in-situ reaction): 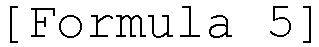 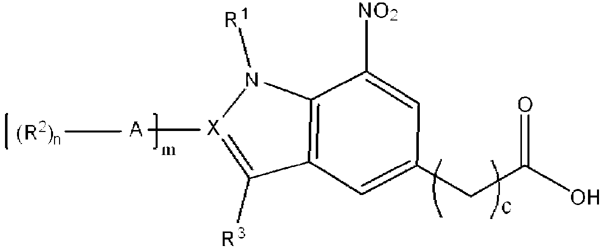 wherein in Formula 5 above, R¹ to R³, A, X, n and m are the same as defined in Formula 1 above, and c is the same as defined in Formula 2 above.

In the present invention, Step b) above which is the linking reaction and Step c) above which is the cyclization reaction for forming indole or indolane can be continuously performed in the same reactor, the intermediate compound formed by reacting the compound represented by Formula 3 above with the compound represented by Formula 4 can be directly reacted with a base without performing a separate work-up or purification process after the synthesis to prepare the compound represented by Formula 5, and even though a separate work-up or purification process is not performed, the compound represented by Formula 5 can be obtained in a high yield, thereby greatly improving the efficiency of the process.

In Step c) above, the base may include, for example, at least one among potassium t-butoxide (KOBu^{t}), potassium carbonate (K₂CO₃), 1,8-diazabicyclo[5.4.0]undec-7-ene (DBU), triethylamine (Et₃N), diisopropylethylamine (EtN(iPr)₂₋), N-methyl morpholine, methyl pyrrolidone, and N-methyl propionamide (NMPA), and preferably, DBU.

In addition, Step c) above may be carried out by adding the base and then raising the temperature to 50-150 °C for 10-30 hours, preferably for 15-20 hours.

In addition, according to an embodiment of the present invention, the preparation method further includes a step (Step d) for adding a reducing agent after Step c) above to prepare the compound represented by Formula 1 above. In this case, Step d) above prepares the compound represented by Formula 1 through a reduction reaction.

In addition, the reducing agent may be at least one among BH₃-DMS (borane dimethylsulfide), NaBH₄, diisobutylaluminum hydride (DIBAL) and lithium aluminum hydride (LAH), and preferably BH₃-DMS (borane dimethylsulfide).

In addition, in Step d) above, the reducing agent may be added at a temperature of 0-30 °C, and specifically, 5-30 °C, or 10-15 °C. In addition, the compound represented by Formula 1 may be synthesized by adding the reducing agent and then raising the temperature to 10-80 °C and stirring for 2-10 hours.

In addition, according to another embodiment of the present invention, the present invention may comprise a method for preparing the compound represented by Formula 6, the method comprising preparing a compound represented by Formula 1 above; and synthesizing the compound represented by Formula 6 below from the compound represented by Formula 1: 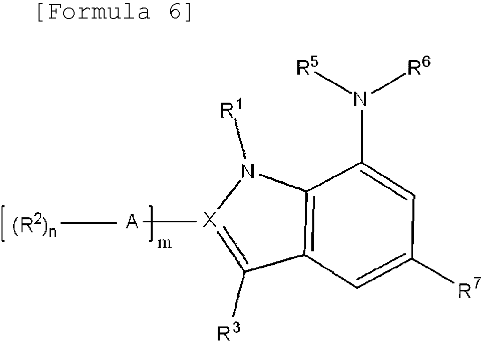 wherein, in Formula 6 above,
R¹, R², R³, n, and m are the same as defined in Formula 1 above,
R⁵ represents hydrogen, C₁-C₆-alkyl, C₃-C₆-cycloalkyl, heterocycle or heterocyclyl-C₁-C₆-alkyl, wherein the heterocycle is a 3-8 membered ring containing one to three heteroatoms selected from among N and O atoms,
R⁶ represents -(CR⁸R⁹)ᵤ-Z-D-W-R¹⁴, wherein u is an integer of 0 to 3, Z represents a direct bond or is selected from the group consisting of -C(O)- and -C(O)O-, D represents a direct bond, C₄-C₆-cycloalkyl, 5-6 membered heteroaryl containing one or two N atoms, or a 5-6 membered heterocycle containing one or two heteroatoms selected from among N, O and S atoms, W represents a direct bond, or -NR⁸-,-C(O)-, - C(O)O-, -C(O)NR¹²- or -S(O)ₜ-, R¹² represents hydrogen, C₁-C₃-alkyl or C₆-C₁₀-aryl, t is an integer of 1 or 2, and R¹⁴ represents hydrogen, hydroxy, C₁-C₆-alkyl, a 5-6 membered heterocycle containing one to three heteroatoms selected from among N, 0 and S atoms, or C₆-C₁₀-Ar-C₁-C₆-alkyl, and
R⁷ represents -Y'R¹¹, wherein Y' is a direct bond or represents -(CR⁸R⁹)ₕY"-, wherein h is an integer of 0 to 3, R¹¹ is selected from the group consisting of hydrogen, halogen, C₁-C₆-alkyl and -(CH₂)ᵥB-R¹³, v is an integer of 0 to 3, B represents a 5-6 membered heterocycle containing one or two heteroatoms selected from among N, O, and S atoms, or represents C₆-C₁₀-aryl, R¹³ represents hydrogen, cyano, halogen, hydroxy, oxo, thiol, carboxy or carboxy-C₁-C₆-alkyl, R⁸ and R⁹ are as defined in claim 1, and Y" is selected from the group consisting of -O-, -C(O)-, and -C(O)O-, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo.

In addition, the method of the present invention may further include a step for purifying after synthesizing each product in Step a), Step c), and Step d), wherein the purification may be performed by a solid purification method.

Specifically, the solid purification according to an embodiment of the present invention may be performed by adding water, a polar organic solvent, or a mixture thereof into a compound synthesized at each step to form a solid, and then filtering and washing the solid. In this case, the polar organic solvent may specifically include at least one among alcohol and dichloromethane, and the alcohol may more specifically include at least one among methanol, ethanol, propanol, and butanol.

In addition, a compound concentrate may be obtained by distillation under reduced pressure before adding the water, the polar organic solvent, or the mixture thereof into the compound, and the method may further include, if necessary, a step for cooling the reactor containing the compound to room temperature (25±5 °C) before the distillation under reduced pressure.

Hereinafter, the present invention will be described in more detail according to the examples.

### Examples

### Example 1: Synthesis of (7-Nitro-2-phenyl-1H-indol-5-yl)methanol

### 1) Synthesis of 4-Amino-3-iodo-5-nitrobenzoic Acid

Commercially available 4-amino-3-nitrobenzoic acid (manufactured by Sinochem Ningbo Co., Ltd. (China)) (5.0 kg), NIS (N-Iodosuccinimide, 9.3 kg), H₂SO₄ (0.50 kg) and THF (25.0 L) were added to a reactor and stirred at room temperature, and then the reactor was heated to a temperature of 80 °C and stirred for 2 hours. In this case, the reaction was terminated when the analysis result of the reaction mixture by HPLC reached 5.0% or less of the peak of 4-amino-3-nitrobenzoic acid.

After confirming the completion of the reaction, the temperature of the reactor was cooled to room temperature, and then dichloromethane (DCM, 50.0 L) was added to a concentrated solution obtained by distillation under reduced pressure to form a solid. The formed solid was stirred at room temperature for 1 hour or more, filtered and washed [1st: DCM (40.0 L), 2nd: H₂O (30.0 L), 3rd: EtOH/H₂O = 3/7, 30.0 L], and then dried under N₂ pressure for 16 hours to synthesize 4-amino-3-iodo-5-nitrobenzoic acid (7.6 kg, Yield: 89.8%, Purity: 96.4%).

1H NMR (500MHz, DMSO-d6) δ 13.1 (br s, 1H), 8.52 (d, J=1.8 Hz, 1H) 8.36 (d, J=1.8 Hz, 1H), 7.50 (s, 2H).

### 2) Synthesis of 7-Nitro-2-phenyl-1H-indole-5-carboxylic Acid

The above 4-amino-3-iodo-5-nitrobenzoic acid (7.6 kg), triethylamine (TEA, 7.5 kg), phenylacetylene (3.0 kg), and 1,4-dioxane (76.0 L) of 1) were added to a reactor and stirred at room temperature, and then CuI [Copper(I) Iodide, 47.0 g] and Pd(PPh₃)₂Cl₂ (173.0 g), which are reaction catalysts, were added thereto, and the reactor was heated to a temperature of 60 °C. The reaction mixture was stirred for 2 hours. Then, the reaction was terminated when the reaction mixture was analyzed by HPLC to show 1.0% or less of the peak of 4-amino-3-iodo-5-nitrobenzoic acid.

Then, 1,8-diazabicyclo[5.4.0]undec-7-ene (DBU, 22.5 kg) was added to the reaction mixture, the reactor was heated to a temperature of 110 °C, the reaction mixture was stirred for 18 hours, and the reaction was then terminated. After the reaction mixture was cooled to room temperature, pH was adjusted to 3 with a 3N-HCl aqueous solution (114.0 L), H₂O (38.0 L) was added thereto, the mixture was stirred for 2 hours or more, filtered and washed [1st: H₂O (38.0 L), 2nd: EtOH/H₂O = 3/7, 23.0 L], and then dried under N₂ pressure for 16 hours to synthesize 7-nitro-2-phenyl-1H-indole-5-carboxylic acid (6.1 Kg, Yield: 87.4%, Purity: 98.0% PAR).

1H NMR (500MHz, DMSO-d6) δ 11.6 (s, 1H), 8.03 (s, 1H), 7.96 (m, 3H), 7.46 (t, J= 7.7 Hz, 2H) 7.36 (d, J= 7.3 Hz, 1H), 7.10 (s, 1H)

### 3) Synthesis of (7-Nitro-2-phenyl-1H-indol-5-yl)methanol

The above 7-nitro-2-phenyl-1H-indole-5-carboxylic acid (5.8 kg) and tetrahydrofuran (THF, 61 L) of 2) were added to a reactor and stirred at room temperature, and then the reaction mixture was cooled to 12±2 °C, and 5M borane dimethylsulfide (BH₃-DMS, 12.3 L) was slowly added dropwise thereto so that the internal temperature thereof did not exceed 40 °C. After the completion of the dropwise addition, the reactor was heated to a temperature of 40 °C, the reaction mixture was stirred for 2 hours, and then the reaction was terminated when the reaction mixture was analyzed by HPLC to show 1.0% or less of the peak of 7-nitro-2-phenyl-1H-indole-5-carboxylic acid.

After confirming the completion of the reaction, H₂O (55 L) was slowly added dropwise to the reaction mixture, and then the mixture was separated into layers to store an organic layer, and the water layer was extracted with ethyl acetate (EtOAc, 29 L), mixed with the stored organic layer, and distilled under reduced pressure. An ethanol/water mixed solvent (ethanol: 6.1 L/water: 12.2 L) was added to the obtained concentrated solution, stirred for 2 hours or more, and then filtered. Then, the resultant was washed with ethanol/water mixed solvent (EtOH/H₂O = 1/3, 9.0 L) as a washing solution and then dried under N₂ pressure for 16 hours to synthesize (7-nitro-2-phenyl-1H-indol-5-yl)methanol (4.6 kg, yield: 83.5%, purity: 98.4%).

1H NMR (500MHz, DMSO-d6) δ 11.6 (s, 1H), 8.07 (s, 1H), 8.00 (m, 3H), 7.49 (t, J= 7.7 Hz, 2H) 7.40 (d, J= 7.3 Hz, 1H), 7.14 (s, 1H), 5.42 (t, J= 6.7 Hz, 1H), 4.66 (d, J= 6.7 Hz, 2H).

## Claims

1. A method for preparing a compound represented by Formula 1, the method comprising:
a) adding a halogen donor to a compound represented by Formula 2 to prepare a compound represented by Formula 3, wherein the halogen donor is N-iodosuccinimide;
b) adding a compound represented by Formula 4 below in the presence of a metal catalyst after Step a) above;
c) adding a base in the presence of the metal catalyst to prepare a compound represented by Formula 5 below;
d) adding a reducing agent to the compound represented by Formula 5 to prepare the compound represented by Formula 1, 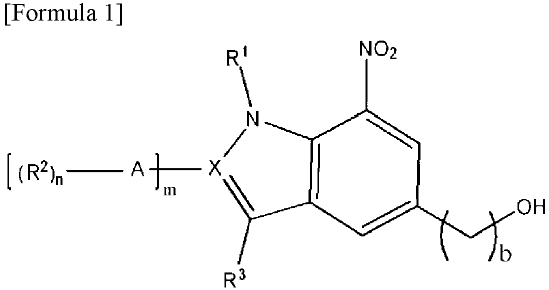 wherein, in Formula 1 above,
n is an integer of 1 to 3,
m is 1,
b is an integer of 1 to 3,
A represents phenyl, or 5-membered heteroaryl or heterocycle which each contains 1 to 3 heteroatoms selected from among N, O and S atoms and is optionally substituted by R, wherein R represents hydrogen or C₁-C₄-alkyl optionally substituted by hydroxy or amino,
X represents C,
R¹ represents hydrogen, C₁-C₆-alkyl or -(CH₂)ᵣNR⁸R⁹, wherein r is an integer of 2 to 5, R⁸ and R⁹ each independently represent hydrogen or C₁-C₃-alkyl,
R² represents hydrogen, halogen or C₁-C₆-alkoxy, or represents -(CH₂)ₚC(O)₂R⁸, -(CH₂)ₚOR⁸, -(CH₂)ₚNR⁸R⁹, -NHR¹⁰, -N(H)S(O)₂R⁸, or -NHC(O)₂R¹⁰, or represents - (CH₂)ₚ-heterocycle-R¹⁰ in which the heterocycle moiety is a 5-6 membered ring containing one or two heteroatoms selected from among N, O, and S atoms, wherein p is an integer of 0 to 3, R⁸ and R⁹ are as defined above, and R¹⁰ represents hydrogen, oxo, C₁-C₆-alkylcarbonyl, C₁-C₆-alkoxy or C₁-C₆- alkyl, or represents a 5-6 membered heterocycle containing one or two nitrogen atoms as a heteroatom, and
R³ represents hydrogen, halogen, C₁-C₆-alkyl or phenyl, or represents -(CH₂)_{q}-heterocycle in which the heterocycle moiety is a 5-6 membered ring containing one or two heteroatoms selected from among N and O atoms, wherein q is an integer of 1 to 3,
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo, 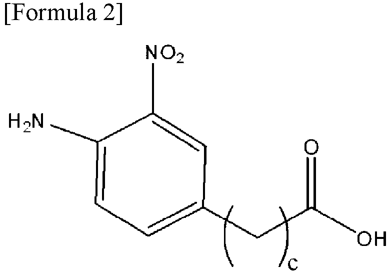
wherein, in Formula 2 above,
c is an integer of 0 to 2, 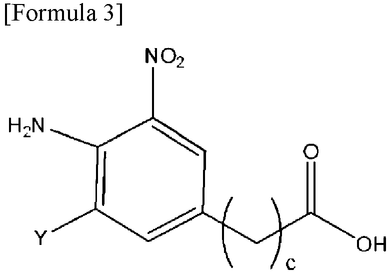
wherein, in Formula 3 above, Y is iodo, and
c is the same as defined in Formula 2 above, 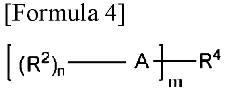
wherein, in Formula 4 above,
R², A, n and m are the same as defined in Formula 1, and
R⁴ is acetylene 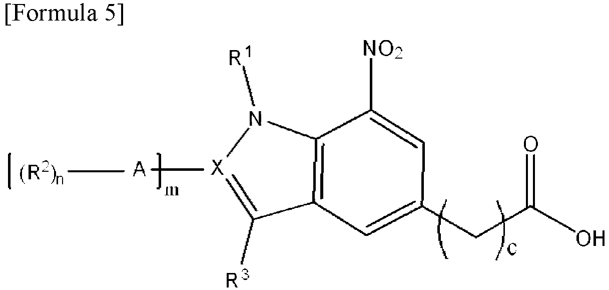
wherein in Formula 5 above, R¹ to R³, A, X, n and m are the same as defined in Formula 1 above, and c is the same as defined in Formula 2 above.

2. The method of claim 1, wherein the metal catalyst is Cu(I) or Pd(II).

3. The method of claim 1, wherein Step b) above and Step c) above are carried out in the same reactor.

4. The method of claim 1, wherein the base comprises at least one selected from the group consisting of potassium t-butoxide (KOBu^{t}), potassium carbonate (K₂CO₃), 1,8-diazabicyclo[5.4.0]undec-7-ene (DBU), triethylamine (Et₃N), diisopropylethylamine (EtN(iPr)₂₋), N-methylmorpholine, methyl pyrrolidone, and N-methylpropionamide (NMPA).

5. The method of claim 1, wherein the compound represented by Formula 1 above is represented by Formula (1a) below: 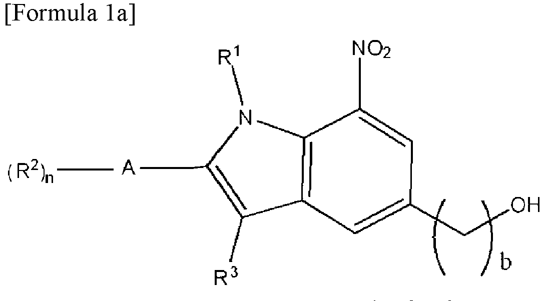 wherein, in Formulae above, n, A, R¹, R², R³ and b are as defined in claim 1.

6. The method of claim 1, wherein the compound represented by Formula 1 above is (7-nitro-2-phenyl-1H-indol-5-yl)methanol, and the compound represented by Formula 2 above is 4-amino-3-nitrobenzoic acid.

7. A method for preparing a compound represented by Formula 6, the method comprising:
preparing a compound represented by Formula 1 according to claim 1; and
synthesizing the compound represented by Formula 6 below from the compound represented by Formula 1, 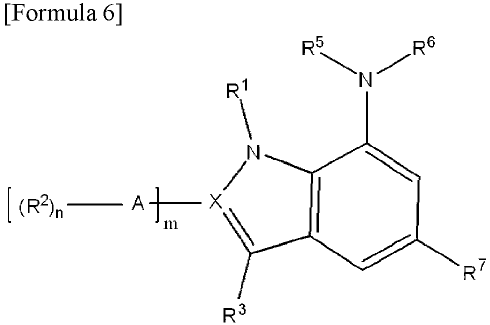
wherein, in Formula 6 above,
R¹, R², R³, n, and m are the same as defined in Formula 1 above,
R⁵ represents hydrogen, C₁-C₆-alkyl, C₃-C₆-cycloalkyl, heterocycle or heterocyclyl-C₁-C₆-alkyl, wherein the heterocycle is a 3-8 membered ring containing one to three heteroatoms selected from among N and O atoms,
R⁶ represents -(CR⁸R⁹)u-Z-D-W-R¹⁴, wherein u is an integer of 0 to 3, Z represents a direct bond or is selected from the group consisting of -C(O)- and -C(O)O-, D represents a direct bond, C₄-C₆-cycloalkyl, 5-6 membered heteroaryl containing one or two N atoms, or a 5-6 membered heterocycle containing one or two heteroatoms selected from among N, O and S atoms, W represents a direct bond, or -NR⁸-,-C(O)-, -C(O)O-, - C(O)NR¹²- or -S(O)t-, R¹² represents hydrogen, C₁-C₃-alkyl or C₆-C₁₀-aryl, t is an integer of 1 or 2, and R¹⁴ represents hydrogen, hydroxy, C₁-C₆-alkyl, a 5-6 membered heterocycle containing one to three heteroatoms selected from among N, 0 and S atoms, or C₆-C₁₀-Ar-C₁-C₆-alkyl, and
R⁷ represents -Y'R¹¹, wherein Y' is a direct bond or represents -(CR⁸R⁹)ₕY"-, wherein h is an integer of 0 to 3, R¹¹ is selected from the group consisting of hydrogen, halogen, C₁-C₆-alkyl and -(CH₂)ᵥB-R¹³, v is an integer of 0 to 3, B represents a 5-6 membered heterocycle containing one or two heteroatoms selected from among N, O, and S atoms, or represents C₆-C₁₀-aryl, R¹³ represents hydrogen, cyano, halogen, hydroxy, oxo, thiol, carboxy or carboxy-C₁-C₆-alkyl, R⁸ and R⁹ are as defined in claim 1, and Y" is selected from the group consisting of -O-, -C(O)-, and -C(O)O-,
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo.

## Patentansprüche

1. Verfahren zur Herstellung einer Verbindung der Formel 1, wobei das Verfahren umfasst:
a) Zugeben eines Halogendonors zu einer Verbindung der Formel 2, um eine Verbindung der Formel 3 herzustellen, wobei der Halogendonor N-Iodosuccinimid ist;
b) Zugeben einer Verbindung der nachstehenden Formel 4 in Gegenwart eines Metallkatalysators nach dem obigen Schritt a);
c) Zugeben einer Base in Gegenwart des Metallkatalysators, um eine Verbindung der nachstehenden Formel 5 herzustellen;
d) Zugeben eines Reduktionsmittels zu der Verbindung der Formel 5, um die Verbindung der Formel 1 herzustellen, 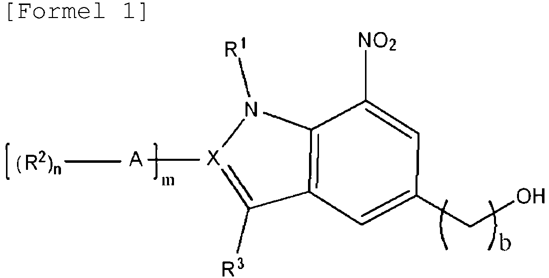 worin in der obigen Formel 1
n eine ganze Zahl von 1 bis 3 ist,
m 1 ist,
b eine ganze Zahl von 1 bis 3 ist,
A Phenyl oder einen 5-gliedrigen Heteroaryl- oder Heterocyclus darstellt, der jeweils 1 bis 3 Heteroatome enthält, die ausgewählt sind aus N-, O- und S-Atomen, und optional durch R substituiert ist, wobei R Wasserstoff oder C₁-C₄-Alkyl darstellt, das optional durch Hydroxy oder Amino substituiert ist,
X C darstellt,
R¹ Wasserstoff, C₁-C₆-Alkyl oder -(CH₂)ᵣNR⁸R⁹ darstellt, wobei r eine ganze Zahl von 2 bis 5 ist, R⁸ und R⁹ jeweils unabhängig Wasserstoff oder C₁-C₃-Alkyl darstellen,
R² Wasserstoff, Halogen oder C₁-C₆-Alkoxy darstellt oder -(CH₂)ₚC(O)₂R⁸, -(CH₂)ₚOR⁸, -(CH₂)ₚNR⁸R⁹ , -NHR¹⁰ , -N(H)S(O)₂R⁸ oder -NHC(O)₂R¹⁰ darstellt oder -(CH₂)ₚ-Heterocyclus-R¹⁰ darstellt, wobei die Heterocyclus-Einheit ein 5- bis 6-gliedriger Ring ist, der ein oder zwei Heteroatome enthält, die ausgewählt sind aus N-, O- und S-Atomen, wobei p eine ganze Zahl von 0 bis 3 ist, R⁸ und R⁹ wie oben definiert sind und R¹⁰ Wasserstoff, Oxo, C₁-C₆-Alkylcarbonyl, C₁-C₆-Alkoxy oder C₁-C₆-Alkyl darstellt oder einen 5- bis 6-gliedrigen Heterocyclus darstellt, der ein oder zwei Stickstoffatome als Heteroatom enthält, und
R³ Wasserstoff, Halogen, C₁-C₆-Alkyl oder Phenyl darstellt oder einen -(CH₂)_{q}-Heterocyclus darstellt, wobei die Heterocyclus-Einheit ein 5- bis 6-gliedriger Ring ist, der ein oder zwei Heteroatome enthält, die ausgewählt sind aus N- und O-Atomen, wobei q eine ganze Zahl von 1 bis 3 ist,
wobei Alkyl, Alkoxy, Aryl, Cycloalkyl, Heterocyclus und Heteroaryl optional substituiert sein können und die Substituenten einen oder mehrere sind, die ausgewählt sind aus der Gruppe bestehend aus Hydroxy, C₁-C₆ -Alkylamino, Di(C₁-C₆-alkyl)amino, Carboxy, C₁-C₆-Alkyl, C₁-C₆-Alkoxy, Carboxy-C₁-C₆-alkyl und Oxo, 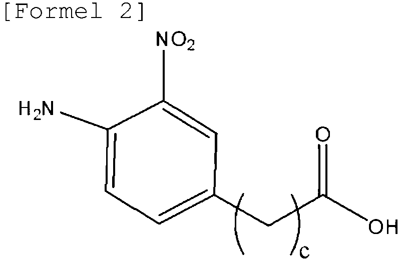
worin in der obigen Formel 2,
c eine ganze Zahl von 0 bis 2 ist, 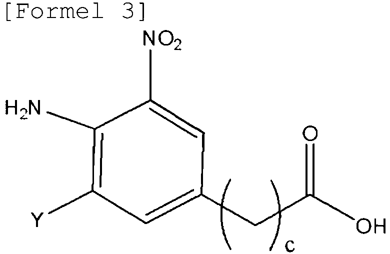
worin in der obigen Formel 3,
Y Iod ist und
c gleich ist, wie in der obigen Formel 2 definiert, 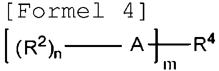
worin in der obigen Formel 4,
R², A, n und m gleich sind wie in Formel 1 definiert und
R⁴ Acetylen ist 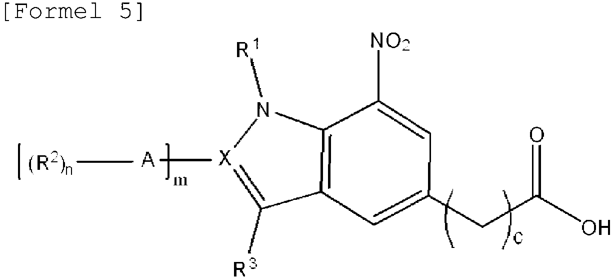
worin in der obigen Formel 5,
R¹ bis R³, A, X, n und m gleich sind wie in der obigen Formel 1 definiert, und c gleich ist wie in der obigen Formel 2 definiert.

2. Verfahren gemäß Anspruch 1, wobei der Metallkatalysator Cu(I) oder Pd(II) ist.

3. Verfahren gemäß Anspruch 1, wobei der obige Schritt b) und der obige Schritt c) in dem gleichen Reaktor durchgeführt werden.

4. Verfahren gemäß Anspruch 1, wobei die Base mindestens eines umfasst, das ausgewählt ist aus der Gruppe bestehend aus Kalium-t-butoxid (KOBu^{t}), Kaliumcarbonat (K₂CO₃), 1,8-Diazabicyclo[5.4.0]undec-7-en (DBU), Triethylamin (Et₃N), Diisopropylethylamin (EtN(iPr)₂₋), N-Methylmorpholin, Methylpyrrolidon und N-Methylpropionamid (NMPA).

5. Verfahren gemäß Anspruch 1, wobei die Verbindung der obigen Formel 1 eine Verbindung der nachstehenden Formel (1a) ist: 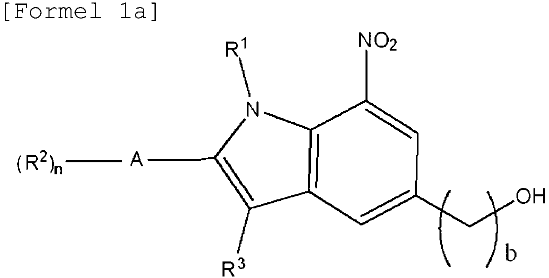 worin in den obigen Formeln n, A, R¹, R², R³ und b wie in Anspruch 1 definiert sind.

6. Verfahren gemäß Anspruch 1, wobei die Verbindung der obigen Formel 1 (7-Nitro-2-phenyl-1H-indol-5-yl)methanol ist und die die Verbindung der obigen Formel 2 4-Amino-3-nitrobenzoesäure ist.

7. Verfahren zur Herstellung einer Verbindung der Formel 6, wobei das Verfahren umfasst:
Herstellen einer Verbindung der Formel 1 gemäß Anspruch 1; und
Synthese der Verbindung der nachstehenden Formel 6 aus der Verbindung der Formel 1, 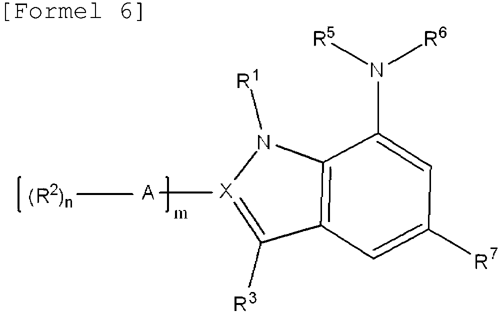 worin in der obigen Formel 6,
R¹, R², R³, n und m gleich sind wie in der obigen Formel 1 definiert,
R⁵ Wasserstoff, C₁-C₆-Alkyl, C₃-C₆-Cycloalkyl, einen Heterocyclus oder Heterocyclyl-C₁-C₆-Alkyl darstellt, wobei der Heterocyclus ein 3- bis 8-gliedriger Ring ist, der ein bis drei Heteroatome enthält, die ausgewählt sind aus N- und O-Atomen,
R⁶ -(CR⁸R⁹)u-Z-D-W-R¹⁴ darstellt, wobei u eine ganze Zahl von 0 bis 3 ist, Z eine direkte Bindung darstellt oder ausgewählt ist aus der Gruppe bestehend aus -C(O)- und -C(O)O-, D eine direkte Bindung, C₄-C₆-Cycloalkyl, ein 5- bis 6-gliedriges Heteroaryl mit einem oder zwei N-Atomen oder einen 5- bis 6-gliedrigen Heterocyclus mit einem oder zwei Heteroatomen darstellt, die ausgewählt sind aus N-, O- und S-Atomen, W eine direkte Bindung oder -NR⁸-, -C(O)-, -C(O)O, -C(O)NR¹²- oder -S(O)t- darstellt, R¹² Wasserstoff, C₁-C₃-Alkyl oder C₆-C₁₀-Aryl darstellt, t eine ganze Zahl von 1 oder 2 ist und R¹⁴ Wasserstoff, Hydroxy, C₁-C₆-Alkyl, einen 5- bis 6-gliedrigen Heterocyclus, der ein bis drei Heteroatome enthält, die ausgewählt sind aus N-, 0- und S-Atomen, oder C₆-C₁₀-Ar-C₁-C₆-Alkyl darstellt, und
R⁷ -Y'R¹¹ darstellt, wobei Y' eine direkte Bindung ist oder -(CR⁸R⁹)ₕY"- darstellt, wobei h eine ganze Zahl von 0 bis 3 ist, R¹¹ ausgewählt ist aus der Gruppe bestehend aus Wasserstoff, Halogen, C₁-C₆-Alkyl und -(CH₂)ᵥB-R¹³, v eine ganze Zahl von 0 bis 3 ist, B einen 5- bis 6-gliedrigen Heterocyclus darstellt, der ein oder zwei Heteroatome enthält, die ausgewählt sind aus N-, O- und S-Atomen, oder C₆-C₁₀-Aryl darstellt, R¹³ Wasserstoff, Cyano, Halogen, Hydroxy, Oxo, Thiol, Carboxy oder Carboxy-C₁-C₆-Alkyl darstellt, R⁸ und R⁹ wie in Anspruch 1 definiert sind, und Y" ausgewählt ist aus der Gruppe bestehend aus -O-, -C(O)- und -C(O)O-,
wobei Alkyl, Alkoxy, Aryl, Cycloalkyl, Heterocyclus und Heteroaryl optional substituiert sein können, und die Substituenten einer oder mehrere sind, die ausgewählt sind aus der Gruppe bestehend aus Hydroxy, C₁-C₆-Alkylamino, Di(C₁-C₆-Alkyl)amino, Carboxy, C₁-C₆-Alkyl, C₁-C₆-Alkoxy, Carboxy-C₁-C₆-Alkyl und Oxo.

## Revendications

1. Procédé de préparation d'un composé représenté par la formule 1, le procédé comprenant :
a) l'ajout d'un donneur d'halogène à un composé représenté par la formule 2 pour préparer un composé représenté par la formule 3, dans lequel le donneur d'halogène est le N-iodosuccinimide ;
b) l'ajout d'un composé représenté par la formule 4 ci-dessous en présence d'un catalyseur métallique après l'étape a) ci-dessus ;
c) l'ajout d'une base en présence du catalyseur métallique pour préparer un composé représenté par la formule 5 ci-dessous ;
d) l'ajout d'un agent réducteur au composé représenté par la formule 5 pour préparer le composé représenté par la formule 1, 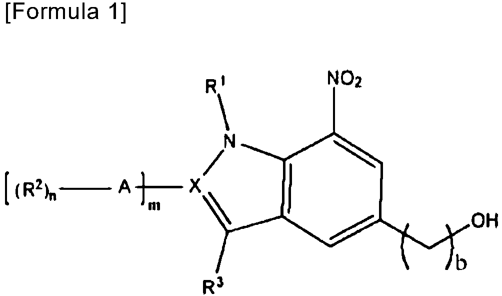 dans lequel, dans la formule 1 ci-dessus,
n est un nombre entier de 1 à 3,
m est 1,
b est un nombre entier de 1 à 3,
A représente un phényle, ou un hétéroaryle ou hétérocycle à 5 chaînons qui contiennent chacun 1 à 3 hétéroatomes sélectionnés parmi les atomes N, O et S et sont facultativement substitués avec R, dans lequel R représente un hydrogène ou un alkyle en C₁-C₄ facultativement substitué avec un hydroxy ou un amino,
X représente C,
R¹ représente un hydrogène, un alkyle en C₁-C₆ ou -(CH₂)ᵣNR⁸R⁹, dans lequel r est un nombre entier de 2 à 5, R⁸ et R⁹ représentent chacun indépendamment un hydrogène ou un alkyle en C₁-C₃,
R² représente un hydrogène, un halogène ou un alcoxy en C₁-C₆, ou représente -(CH₂)ₚC(O)₂R⁸, -(CH₂)ₚOR⁸, -(CH₂)ₚNR⁸R⁹, -NHR¹⁰, -N(H)S(O)₂R⁸, ou -NHC(O)₂R¹⁰, ou représente -(CH₂)ₚ-hetérocycle-R¹⁰ dans lequel le fragment hétérocycle est un cycle à 5 ou 6 chaînons contenant un ou deux hétéroatomes sélectionnés parmi les atomes N, O, et S, dans lequel p est un nombre entier de 0 à 3, R⁸ et R⁹ sont tels que précédemment définis, et R¹⁰ représente un hydrogène, un oxo, un alkylcarbonyle en C₁-C₆, un alcoxy en C₁-C₆ ou un alkyle en C₁-C₆, ou représente un hétérocycle à 5 ou 6 chaînons contenant un ou deux atomes d'azote en tant qu'hétéroatome, et
R³ représente un hydrogène, un halogène, un alkyle en C₁-C₆ ou un phényle, ou représente un -(CH₂)_{q}-hétérocycle dans lequel le fragment hétérocycle est un cycle à 5 ou 6 chaînons contenant un ou deux hétéroatomes sélectionnés parmi les atomes N et O, dans lequel q est un nombre entier de 1 à 3,
dans lequel les alkyle, alcoxy, aryle, cycloalkyle, hétérocycle et hétéroaryle peuvent être facultativement substitués, et les substituants sont un ou plusieurs sélectionnés dans le groupe consistant en un hydroxy, un alkylamino en C₁-C₆, un di(alkyle en C₁-C₆)amino, un carboxy, un alkyle en C₁-C₆, un alcoxy en C₁-C₆, un carboxy-alkyle en C₁-C₆ et un oxo, 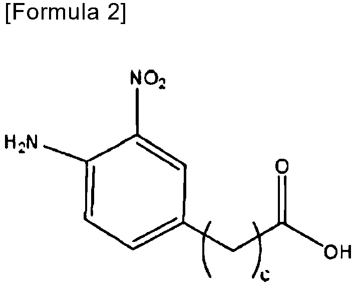
dans lequel, dans la formule 2 ci-dessus,
c est un nombre entier de 0 à 2, 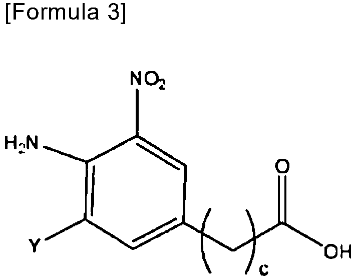
dans lequel, dans la formule 3 ci-dessus, Y est un iodo, et
c est tel que défini dans la formule 2 ci-dessus, 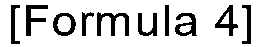 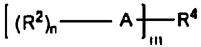
dans lequel, dans la formule 4 ci-dessus,
R², A, n et m sont tels que définis dans la formule 1, et
R⁴ est un acétylène 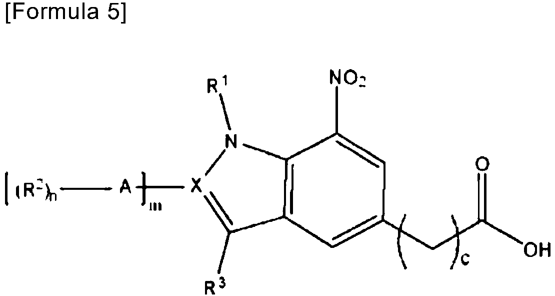
dans lequel dans la formule 5 ci-dessus, R¹ à R³, A, X, n et m sont tels que définis dans la formule 1 ci-dessus, et c est tel que défini dans la formule 2 ci-dessus.

2. Procédé selon la revendication 1, dans lequel le catalyseur métallique est Cu(I) ou Pd(II).

3. Procédé selon la revendication 1, dans lequel l'étape b) ci-dessus et l'étape c) ci-dessus sont mises en œuvre dans le même réacteur.

4. Procédé selon la revendication 1, dans lequel la base comprend au moins un sélectionné dans le groupe consistant en tert-butanolate de potassium (KOBu^{t}), carbonate de potassium (K₂CO₃), 1,8-diazabicyclo[5.4.0]undéc-7-ène (DBU), triéthylamine (Et₃N), diisopropyléthylamine (EtN(iPr)₂₋), N-méthylmorpholine, méthylpyrrolidone, et N-méthylpropionamide (NMPA).

5. Procédé selon la revendication 1, dans lequel le composé représenté par la formule 1 ci-dessus est représenté par la formule (1a) ci-dessous : 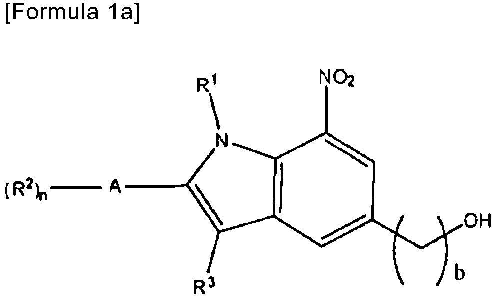 dans lequel, dans les formules ci-dessus, n, A, R¹, R², R³ et b sont tels que définis dans la revendication 1.

6. Procédé selon la revendication 1, dans lequel le composé représenté par la formule 1 ci-dessus est le (7-nitro-2-phényl-1H-indol-5-yl)méthanol, et le composé représenté par la formule 2 ci-dessus est l'acide 4-amino-3-nitrobenzoïque.

7. Procédé de préparation d'un composé représenté par la formule 6, le procédé comprenant :
la préparation d'un composé représenté par la formule 1 selon la revendication 1 ; et
la synthèse du composé représenté par la formule 6 ci-dessous à partir du composé représenté par la formule 1, 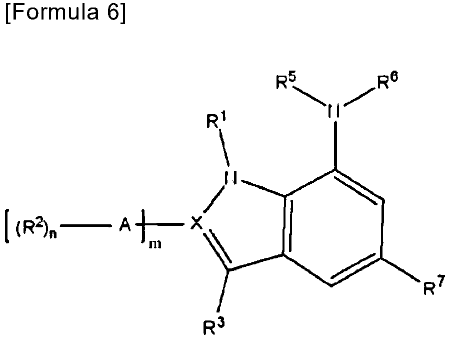
dans lequel, dans la formule 6 ci-dessus,
R¹, R², R³, n, et m sont tels que définis dans la formule 1 ci-dessus,
R⁵ représente un hydrogène, un alkyle en C₁-C₆, un cycloalkyle en C₃-C₆, un hétérocycle ou un hétérocyclyl-alkyle en C₁-C₆, dans lequel l'hétérocycle est un cycle de 3 à 8 chaînons contenant un à trois hétéroatomes sélectionnés parmi les atomes N et O,
R⁶ représente -(CR⁸R⁹)u-Z-D-W-R¹⁴, dans lequel u est un nombre entier de 0 à 3, Z représente une liaison directe ou est sélectionné dans le groupe consistant en - C(O)- et -C(O)O, D représente une liaison directe, un cycloalkyle en C₄-C₆, un hétéroaryle à 5 ou 6 chaînons contenant un ou deux atomes N, ou un hétérocycle à 5 ou 6 chaînons contenant un ou deux hétéroatomes sélectionnés parmi les atomes N, O et S, W représente une liaison directe, ou -NR⁸-, -C(O)-, -C(O)O-, - C(O)NR¹²- ou - S(O)t-, R¹² représente un hydrogène, un alkyle en C₁-C₃ ou un aryle en C₆-C₁₀, t est un nombre entier égal à 1 ou 2, et R¹⁴ représente un hydrogène, un hydroxy, un alkyle en C₁-C₆, un hétérocycle à 5 ou 6 chaînons contenant un à trois hétéroatomes sélectionnés parmi les atomes N, O et S, ou un Ar en C₆-C₁₀-alkyle en C₁-C₆, et
R⁷ représente -y'R¹¹, dans lequel Y' est une liaison directe ou représente - (CR⁸R⁹)ₕY"-, dans lequel h est un nombre entier de 0 à 3, R¹¹ est sélectionné dans le groupe consistant en un hydrogène, un halogène, un alkyle en C₁-C₆ et -(CH₂)ᵥB-R¹³, v est un nombre entier de 0 à 3, B représente un hétérocycle à 5 ou 6 chaînons contenant un ou deux hétéroatomes sélectionnés parmi les atomes N, O, et S,
ou représente un aryle en C₆-C₁₀, R¹³ représente un hydrogène, un cyano, un halogène, un hydroxy, un oxo, un thiol, un carboxy ou un carboxy-alkyle en C₁-C₆, R⁸ et R⁹ sont tels que définis dans la revendication 1, et Y" est sélectionné dans le groupe consistant en -O-, -C(O)-, et -C(O)O-,
dans lequel les alkyle. alcoxy, aryle, cycloalkyle, hétérocycle et hétéroaryle peuvent être facultativement substitués, et les substituants sont un ou plusieurs sélectionnés dans le groupe consistant en un hydroxy, un alkylamino en C₁-C₆, un di(alkyle en C₁-C₆)amino, un carboxy, un alkyle en C₁-C₆, un alcoxy en C₁-C₆, un carboxy-alkyle en C₁-C₆ et un oxo.
